# EUROPEAN PATENT APPLICATION

(11) **EP 2 458 044 A1**
(43) Date of publication of application: **30.05.2012**
(21) Application number: 10192396.9
(22) Date of filing: 24.11.2010
(51) Int. Cl.: D01D 5/00, D01F 1/10, A61L 17/08, A61L 27/24, D06M 11/00, D06M 11/71, B82Y 30/00

(54) **Product containing nanocrystals and method for producing the same**

(71) Applicant: SpinPlant GmbH, 04103 Leipzig (DE)
(72) Inventor: Dr. Ganey, Timothy, Tampa, FL 33604 (US); Prof. Dr. Meisel, Jörg, Berlin, 14163 (DE)
(74) Representative: Sokolowski, Fabian

(57) **Abstract**

The invention relates to a product comprising a carrier material being built up from nanofibers having a diameter of less than 1200 nm. According to the invention, polarized or charged nanocrystals of at least one first auxiliary substance are chemically and/or physically bound to the carrier material. The invention further relates to methods for producing such a product.

## Description

The invention relates to a product of nanofibers according to the preamble of claim 1, to methods for producing such a product according to the preambles of claims 8 and 9 and the use of such a product according to claim 14.

From prior art, many basic materials for bone substituents or suture materials are known. Some of these materials consist of nanofibers. However, many materials do not promote tissue adhesion or growth in a sufficiently satisfactory way.

It is an object of the invention to provide a product which enhances tissue growth and/or tissue adhesion better than products known from prior art.

This object is achieved by a product having the features of claim 1. Such a product comprises a carrier material being built up from nanofibers having a diameter of less than 1200 nm. According to the invention, polarized or charged nanocrystals of at least one first auxiliary substance are chemically and/or physically bound to the carrier material. By those nanocrystals, the nanofibers can exhibit a long-lasting conductive effect which enhances tissue growth and/or adhesion along the nanofibers. Accordingly, a product being built up from such nanofibers containing nanocrystals is well suited for promoting tissue adhesion and/or growth.

In an embodiment, the nanofibers might have a diameter of less than 1 100 nm, in particular less than 1 000 nm, in particular less than 900 nm, in particular less than 800 nm, in particular less than 700 nm, in particular less than 600 nm, in particular less than 500 nm, in particular less than 400 nm, in particular less than 300 nm.

The nanocrystals may mediate and/or promote conductive or semi-conductive properties of the carrier material, i.e. the material being built up from the nanofibers. Additionally, nanofibers containing polarized nanocrystals can exhibit a piezoelectric effect and may thus be used as voltage supply upon deformation. Thus, the product according to the invention may be also used as supply for electrical power which is generated upon exerting pressure onto the product.

The use of polarized nanocrystals is particularly preferred. The polarized nanocrystals may also be denoted as nanocapacitors. They exhibit polarity and thus at least two electric poles being separated from each other in the crystal.

A very well method for obtaining nanofibers is electrospinning. Electrospun nanofibers already carry with them a capacitive voltage that is polarized. This capacitive voltage is enhanced and made long-lasting by the addition of nanocrystals. Electrospun nanofibers are very well suited for building up piezofabrics. Extrusion, especially by using tension polymerization is also a method by which according nanofibers may be produced.

In an embodiment, the first auxiliary substance is chosen from the group consisting of osteoinductive substances, electrically conductive substances, electrically semiconductive substances, electrically insulating substances, antibacterial substances, antiviral substances, antifungal substances, ceramics (like, e.g., hydroxyapatite ceramics which are particularly well suited for forming nanocrystals), barium, bromine, copper, niobium, lithium, germanium, titanium, lead, zirconium, silicon, silver, zinc, polyurethane, silver hydrogen sulfate, gallium orthophosphate (GaPO₄), langasite (La₃Ga₅SiO₁₄), barium titanate (BaTiO₃), lead titanate (PbTiO₃), lead zirconate titanate (Pb[ZrₓTi₁₋ₓ]O₃ 0<x<1), potassium niobate (KNbO₃), lithium niobate (LiNbO₃), lithium tantalate (LiTaO₃), sodium tungstate (Na₂WO₃), Ba₂NaNb₅O₅ and Pb₂KNb₅O₁₅.

Not all of these substances might be primarily suited for use within a human or animal body. They might be used, however, in a product to be used for enhancing tissue growth in vitro. Furthermore, in case of the product being a microlaminar voltaic in which the auxiliary substances are sealed to be for example used in battery stacks, also apparently toxic members of the group of the first auxiliary substance are well suited to build up the nanocrystals to be used.

In a further embodiment, the carrier material is solved or dispersed in at least one liquid chosen from the group of water, alcohols like methanol or ethanol, aqueous solutions of acids or bases like acetic acid or sodium hydroxide and organic solvents like acetone or 1,1,1,3,3,3-hexaflouoro-2-propanol to produce a carrier material solution.

The term "carrier material solution" also encompasses "carrier material dispersions". This means, it is not necessary that the carrier material is ideally solved in an according liquid. If an essentially stable dispersion of the carrier material in an according liquid is established, electrospinning can also take place.

In a further embodiment, the carrier material is collagen, a mixture of collagen and hydroxy apatite, gelatin, alginates, chitosan, silk, cellulose, polyurethane, a polyester, polycaprolactone, polylactide, polypyrrole, polyaniline, polyacetylene, polythiophene, a copolymer of the preceding polymers and/or a copolymer bearing carboxylic acid groups and/or amine groups.

Further suited carrier materials are amino acid structures like oligopeptides or polypeptides. Oligopeptides are considered to consist of a sequence of up to 10 amino acids, whereas polypeptides are considered to be amino acid structures having more than 10 amino acids. Proteins are to be considered to be encompassed by the term "polypeptide". This means the amino acids structure can exhibit a primary, secondary and tertiary structure by itself so that the structure of the material to be deposited on the collector can exhibit an individually adjusted sub-structure. By using an amino acid structure, one can take advantage not only of the secondary or tertiary structure of the oligopeptides or polypeptides, but also of the amphiphilic charge of the single amino acids being present in the oligopeptides or polypeptides.

Well-suited collagens are collagen type I, II, III, V, or XI, wherein type I collagen is particularly well suited. The collagen might for example have a human, bovine, equine, ovine or fish origin or can be an artificial collagen resembling human, bovine, equine, ovine or fish collagen, or might consist of collagen, or collagen fibrils that have been expressed in culture by vector incorporation without limitation to mammalian source.

In a further embodiment, the carrier material comprises collagen and a crosslinker. In this case, the product consists of a carrier material which is biodegradable. Such a product can be implanted into a human or animal body and is degraded overtime so that no subsequent removal of the product is necessary. Such a product can for example serve as temporary bone substitute. In doing so, it enhances bone growth along the product and reduces the necessary time for healing processes.

The term "crosslinker" encompasses chemical substances which crosslink the carrier material by forming chemical bonds to the carrier material and/or by forming a polymer in which the carrier material is embedded. It is possible that chemical bond formation between crosslinker and carrier material also takes place in case of a polymer of crosslinker in which the carrier material is embedded. Thus, the carrier material can intercalate a polymer of a crosslinker and additionally can form chemical bonds to the crosslinker. Alternatively, only chemical bond formation (without polymeric embedding or intercalation) can take place. A substance comprising a polymer of a crosslinker and an embedded (or embedded and at least partially chemical linked) carrier material can also be denoted as composite or as nanofiber reinforced composite.

In a further embodiment, the product also comprises at least one second auxiliary substance. This second auxiliary substance is chosen from the group consisting of osteoinductive substances, electrically conductive substances, electrically semiconductive substances, electrically insulating substances, antibacterial substances, antiviral substances, antifungal substances, ceramics (like, e.g. hydroxyapatite ceramics), barium, bromine, copper, niobium, lithium, germanium, titanium, lead, zirconium, silicon, silver, zinc, polyurethane, silver hydrogen sulfate. In contrast to the first auxiliary substance, the second auxiliary substance is present in a non-nanocrystalline form in the product. It is - as is a first auxiliary substance - chemically and/or physically bound to the carrier material.

By combining the nanocrystalline first auxiliary substance and a non-nanocrystalline second auxiliary substance, the nanocapacitive properties of polarized nanocrystals as well as the general properties of the substances belonging to the group of the second auxiliary substance can be combined in a particular advantageous manner. For example, an osteoinductive material can be applied to the product in a non-nanocrystalline form, wherein a conductive material can be applied in a nanocrystalline form. The product then exhibits both osteoinductive properties as well as electrically conductive properties. This combination enhances tissue growth and/or cell adhesion to the product in a particularly advantageous manner.

The first and/or the second auxiliary substance can be entrapped within the polymer network of the carrier material or the carrier material and the crosslinker, respectively. The entrapment offers the effect that a certain placement/precipitation of the auxiliary substance can be achieved, e.g. a placement/precipitation in the banding structure of the carrier material perpendicular to scaffold orientation. Thus, if hydroxyapatite is used as auxiliary substance and collagen is used as carrier material, the natural orientation of hydroxyapatite with respect to collagen in normal bone (i.e. in the banding structure of bone perpendicular to scaffold orientation) can be mimicked.

In an embodiment, the product exhibits an maximum tensile strength of ca. 50 to 200 MPa, in particular of ca. 75 to 175 MPa, in particular of ca. 90 to 150 MPa, in particular of ca. 100 to 125 MPa.

In a further embodiment, the product exhibits an elastic modulus of ca. 300 to 700 MPa, in particular of ca. 350 to 650 MPa, in particular of ca. 400 to 600 MPa, in particular of ca. 450 to 580 MPa, in particular of ca. 500 to 550 MPa.

The object is also achieved by a method for producing a product according to the preceding explanations comprising the steps of providing a carrier material solution comprising a carrier material, and bringing the carrier material in contact with a collector by electrospinning the carrier material solution out of a spinning device. Thereby, the collector has a first electrical polarity and the spinning device has a second electrical polarity which is opposite to the first polarity. According to the invention, the carrier material solution also comprises charged or polarized nanocrystals of at least one first auxiliary substance so that the nanocrystals are electrospun together with the carrier material and a chemically and/or physically bound to the carrier material after electrospinning.

This means that according to this method, both carrier material solution and polarized or charged nanocrystals are electrospun together so as to form nanofibers onto which at least one first auxiliary substance is chemically and/or physically bound in form of nanocrystals. By adjusting the concentration of the first auxiliary substance in the carrier material solution, the average concentration of the nanocrystals in the electrospun nanofibers can be determined.

The spinning device may be a nozzle through which the carrier material solution can be sprayed, or a turning (or rotating) pin located in a bath of the carrier material solution. By turning or rotating the pin in the bath, the carrier material solution is transported out of the bath and accelerated in the direction of the collector.

In order to carry out this method, it is advantageous if the nanocrystals can be dispersed equally in the carrier material solution essentially without being separated from the carrier material solution after being present in it for a long while. However, even if a certain separation would take place, a mixing of the carrier material solution by stirring the solution or by using a rotating pin to spin a part of the solution out of the reservoir containing the solution, a re-dispersion can be achieved.

In cases in which a stable dispersion of polarized nanocrystals in the carrier material solution cannot be established (this can be due to the chemical and/or physical behaviour of either the carrier material solution or the polarized nanocrystals), another method for producing a product according to the above explanations can be carried out.

According to this alternative method, a carrier material solution comprising a carrier material is provided. Afterwards, the carrier material is brought into contact with a collector by electrospinning the carrier material solution out of a spinning device, wherein the collector has a first electrical polarity and the spinning device has a second electrical polarity which is opposite to the first polarity. Subsequently, polarized or charged nanocrystals of at least one first auxiliary substance are applied to the already electrospun carrier material to be chemically and/or physically bound to the carrier material. This means, in this alternative method, electrospun fibers are already present when the nanocrystals of the at least one first auxiliary substance are applied to the nanofibers. This means that the nanocrystals are primarily present on the surface of the electrospun nanofibers.

In contrast, when carrying out the first method explained above, the nanocrystals are not only present on the surface of the nanofibers, but also in their interior because both nanocrystals and carrier materials are spun together to nanofibers.

For example, an ionic carrier material might be used and electrospun to nanofibers. Then, charged nanofibers result. If afterwards charged nanocrystals are applied to those charged nanofibers, the charge of the nanofibers can be enhanced, reduced or neutralized - depending on the charge of the nanocrystals. If nanocapacitors are used as polarized nanocrystals, the charge of the carrier material will not be influenced since those nanocapacitors are per se neutral. If, however, charged nanocrystals are used, a highly resolved ionic surface can be printed onto the nanofibers.

In an embodiment, either the first or the second variant of the method may be carried out by performing the electrospinning at a voltage of 8 to 20 kV, in particular 10 to 17 kV, in particular 12 to 15 kV between the collector and the spinning device. A voltage of approximately 12.5 kV is particularly suited. Those voltages are well suited for accelerating the carrier material solution sufficiently fast out of the spinning device towards the collector. Further electrospinning parameters like the speed of a rotating pin for ejecting some of the carrier material solution out of a reservoir of the carrier material solution or the flow rate of the carrier material solution towards a nozzle, and the distance between the spinning device and the collector can be adjusted to the respective needs according to standard protocols of electrospinning.

In a further embodiment, the carrier material alone or the carrier material together with the nanocrystals is deposited onto the collector to form a sheet material. Such a sheet material can be used as bone substitute material or as wound-covering fleece.

In an alternative embodiment, the carrier material alone or the carrier material together with the nanocrystals is removed from the collector after having contacted it and is then spun to a yarn. Such a yarn may be used as suture material in orthopaedic or surgical applications. The linear material, or yarn, or bioyarn, may also be embodied as a raw material characterized by its strength, stiffness, elasticity, modulus, charge and composition as to make it a linear material that can be braided, woven, knitted, plied, or in other ways converted to mesh, fabric, matted material, or laminated with specific endowment as to afford areas between the threads to dynamic as well as static properties that align to the degradation of the material and regeneration of the biologic tissue.

If the carrier material solution is electrospun without having been augmented with nanocrystals, the nanocrystals are applied to the carrier material in an embodiment of the invention before the carrier material is spun to a yarn. This means that in this embodiment nanofibers are spun together with nanocrystals to a yarn so that the nanocrystals are present within the interior of the yarn and not only on the exterior of the yarn which would be the case if the nanocrystals would only be applied after the yarn is already spun. Thereby, a more uniform distribution of nanocrystals within the yarn can be achieved as compared to applying the naoncrystals only after having spun the yarn already. An even more uniform distribution of the nanocrystals within the carrier material can be achieved by electrospinning both carrier material solution and nanocrystals together to nanofibers and then spinning those nanofibers already containing nanocrystals further to a yarn.

In a further embodiment, the electrospun carrier material is washed after having been removed from the collector. By washing, remainders of non-reacted crosslinker or reaction co-products can be removed, thus improving the biocompatibility of the obtained product. Washing can be performed by an aqueous solution of an alcohol like for example ethanol in a concentration of for example 80 %, 70 %, 60 % or 50 % (v/v). Further suited washing solutions are low salt buffers like phosphate buffered saline (PBS). Washing can be performed in a two-step manner by first using an alcoholic solution and afterwards a low salt buffer (or first a low salt buffer and afterwards an alcoholic solution) as washing solutions.

In an alternative embodiment, no washing step is necessary due to the specific crosslinker chosen and the concentration in which it is used.

Further embodiments explained with respect to the claimed product can also be applied in an analogous way to the claimed method and are not repeated here for the sake of brevity only.

A product having the characteristics as explained above can be very well used as scaffold for growing cells in vitro or in vivo or as suture material. The cells to be grown can be mesenchymal stem cells, fully competent stem cells, expanded somatic lineages, separated and suspended cells, peripheral circulating cells and cells of either included or induced potential. In vivo cell growth can be for example achieved with respect to bone growth or with respect to healing processes of wounds. Suture material made of a product as explained above can also ameliorate healing processes at the suture sites since cells attach more easily to such a suture material than to conventional suture materials.

For example, the sheet material can be implanted into a body of a patient (either human or animal) and act as (temporary) bone substitute material. If it is made from a biodegradable or bioresorbable material like for example collagen, the sheet material will be resorbed or degraded over time and newly grown bone will replace the sheet material step by step. By promoting bone growth due to the specific structure of the sheet material, healing processes after operations are accelerated. The use of a sheet material as described above for enabling tissue growth either in vivo or in vitro is also part of the invention. Specifically, the use of a sheet material as described above as bone substitute material is encompassed by the invention. The sheet material may embody itself with charge that potentiates tissue differentiation. Stem cell differentiation has been closely tied to electrovolt membrane potential during phenotypic emergence. Electrospinning, charge potentiation, and multi-laminar sheet formation all carry the option to defined physical conditions of the matrix, and define voltage differences in regenerative tissues. Processed materials from electrospun biologic components, both linear and in piled matte, will be used to define matrix charge and enliven the differentiation process.

## Claims

1. Product comprising a carrier material being built up from nanofibers having a diameter of less than 1200 nm, **characterized in that** polarized or charged nanocrystals of at least one first auxiliary substance are chemically and/or physically bound to the carrier material.

2. Product according to claim 1, **characterized in that** the nanofibers are obtained by electrospinning.

3. Product according to claim 1 or 2, **characterized in that** the nanocrystals have a mean diameter in the range of 10 to 200 nm.

4. Product according to any of the preceding claims, **characterized in that** the first auxiliary substance is chosen from the group consisting of osteoinductive substances, electrically conductive substances, electrically semiconductive substances, electrically insulating substances, antibacterial substances, antiviral substances, antifungal substances, ceramics, barium, bromine, copper, niobium, lithium, germanium, titanium, lead, zirconium, silicon, silver, zinc, polyurethane, silver hydrogen sulfate, gallium orthophosphate, langasite, barium titanate, lead titanate, lead zirconate titanate, potassium niobate, lithium niobate, lithium tantalate, sodium tungstate, Ba₂NaNb₅O₅ and Pb₂KNb₅O₁₅.

5. Product according to any of the preceding claims, **characterized in that** the carrier material comprises collagen, a mixture of collagen and hydroxy apatite, gelatin, alginates, chitosan, silk, cellulose, polyurethane, a polyester, polycaprolactone, polylactide, polypyrrole, polyaniline, polyacetylene, polythiophene, a copolymer of the preceding polymers and/or a copolymer bearing carboxylic acid groups and/or amine group, as well as oligopeptides or polypeptides.

6. Product according to any of the preceding claims, **characterized in that** the carrier material comprises collagen and a crosslinker.

7. Product according to any of the preceding claims, **characterized in that** it further comprises at least one second auxiliary substance of the group consisting of osteoinductive substances, electrically conductive substances, electrically semiconductive substances, electrically insulating substances, antibacterial substances, antiviral substances, antifungal substances, ceramics, barium, bromine, copper, niobium, lithium, germanium, titanium, lead, zirconium, silicon, silver, zinc, polyurethane, silver hydrogen sulfate, wherein the second auxiliary substance is chemically and/or physically bound to the carrier material in a non-nanocrystalline form.

8. Method for producing a product according to any of the preceding claims, comprising the following steps:
- providing a carrier material solution comprising a carrier material,
- bringing the carrier material in contact with a collector by electrospinning the carrier material solution out of a spinning device, the collector having a first electrical polarity and the spinning device having a second electrical polarity being opposite to the first polarity,
**characterized**
**in that** the carrier material solution also comprises polarized or charged nanocrystals of at least one first auxiliary substance so that the nanocrystals are electrospun together with the carrier material and are chemically and/or physically bound to the carrier material after electrospinning.

9. Method for producing a product according to any of claims 1 to 7, comprising the following steps:
- providing a carrier material solution comprising a carrier material,
- bringing the carrier material in contact with a collector by electrospinning the carrier material solution out of a spinning device, the collector having a first electrical polarity and the spinning device having a second electrical polarity being opposite to the first polarity,
**characterized**
**in that** polarized or charged nanocrystals of at least one first auxiliary substance are applied to the electrospun carrier material to be chemically and/or physically bound to the carrier material.

10. Method according to claim 8 or 9, **characterized in that** the electrospinning is done at a voltage of 8 to 20 kV between the collector and the spinning device.

11. Method according to any of claims 8 to 10, **characterized in that** the carrier material or the carrier material and the nanocrystals are deposited onto the collector to form a sheet material.

12. Method according to any of claims 8 to 10, **characterized in that** the carrier material or the carrier material and the nanocrystals are removed from the collector after having contacted it and are then spun to a yarn.

13. Method according to claims 9 and 12, **characterized in that** the nanocrystals are applied to the carrier material before it is spun to a yarn.

14. Use of a product according to any of claims 1 to 7 as scaffold for growing cells in vitro or as suture material.
